# EUROPEAN PATENT APPLICATION

(11) **EP 3 767 635 A1**
(43) Date of publication of application: **20.01.2021**
(21) Application number: 20185685.3
(22) Date of filing: 14.07.2020
(51) Int. Cl.: G16H 40/20, G16H 40/67, H04L 29/08, A61B 5/00, G06Q 50/00

(54) **DATA CAPTURE FROM DISPARATE MEDICAL DEVICES**

(30) Priority: 15.07.2019 US 201962874147 P
(71) Applicant: Hill-Rom Services, Inc., Batesville, IN 47006-9167 (US)
(72) Inventor: CHAHAL, Jotpreet, Batesville, IN 47006-9167 (US); FITZGIBBONS, Stacey A., Batesville, IN 47006-9167 (US); MEYERSON, Craig M., Batesville, IN 47006-9167 (US); ZAPFE, Lori Ann, Batesville, IN 47006-9167 (US)
(74) Representative: Loustalan, Paul William

(57) **Abstract**

A gateway device for connecting a medical device to an electronic medical record can include: a wireless radio to connect with a medical device positioned within a location of a patient; a processor; and memory encoding instructions which, when executed by the processor, cause the gateway device to: allow a caregiver to select the medical device for connection; identify a type of the medical device; associate the medical device with the patient; facilitate delivery of medical data from the medical device to the electronic medical record associated with the patient using the wireless radio; present a plurality of algorithms for configuring the medical device to the caregiver for selection; and present a plurality of data visualizations for visualizing the medical data captured by the medical device to the caregiver for selection.

## Description

### INTRODUCTION

In a complex acute clinical environment, data is often collected by caregivers at various points throughout the day and not always entered into the electronic medical record (EMR) in a timely manner. These delays can cause caregivers to miss subtle changes in a patient's condition or alert on some conditions hours after their onsets.

### SUMMARY

In one aspect of the present disclosure, an example gateway device for connecting a medical device to an electronic medical record includes: a wireless radio to connect with a medical device positioned within a location of a patient; a processor; and memory encoding instructions which, when executed by the processor, cause the gateway device to: allow a caregiver to select the medical device for connection; identify a type of the medical device; associate the medical device with the patient; facilitate delivery of medical data from the medical device to the electronic medical record associated with the patient; present a plurality of algorithms for configuring the medical device to the caregiver for selection; and present a plurality of data visualizations for visualizing the medical data captured by the medical device to the caregiver for selection.

These and other aspects and embodiments are described in detail below, in relation to the attached drawing figures.

### BRIEF DESCRIPTION OF THE DRAWINGS

FIG. 1 is a schematic diagram of a system for receiving data from various medical devices in a care location.
FIG. 2 is an example user interface that allows for the selection and configuration of a new medical device introduced into the care location of FIG. 1.
FIG. 3 illustrates a method of configuring and acquiring data from medical devices within the care location of FIG. 1.
FIG. 4 illustrates example physical components of a computing device of the devices of FIG. 1.

### DETAILED DESCRIPTION

Various embodiments and advantages are explained more fully with reference to the non-limiting examples that are described and illustrated in the accompanying drawings and detailed in the following description. The features illustrated in the drawings are not necessarily drawn to scale, and features of one embodiment may be employed with other embodiments, even if not explicitly stated herein.

The examples used herein are intended merely to facilitate an understanding of ways in which the claimed subject matter may be practiced and to enable those of skill in the art to practice the embodiments of the claimed subject matter described herein. The embodiments provided herein are merely illustrative and should not be construed as limiting the scope of the claimed subject matter, which is defined solely by the appended claims. Also, like reference numerals may represent similar parts throughout the several views of the drawings.

The present disclosure describes a system in which disparate medical devices at the location of care are interconnected to allow for the timely, efficient aggregation of medical data.

In the examples provided herein, the system can detect compatible devices and communicate directly to each other to ensure timely, bedside aggregation of data. At initial set-up, a gateway device will detect which compatible devices are in the patient room and recommend which algorithms and visualizations would be available for use with those devices.

Once set up, each time the caregiver is at the bedside, it will use the configuration selected. When there are changes to available devices at the location, the caregiver can be notified either remotely or when the caregiver arrives at the bedside. The caregiver and devices may be located using Bluetooth (e.g., BLE) or other communication schemes, such as the real-time location system (RTLS) from Hillrom of Batesville, Indiana or millimeter wave. Some example medical devices include vital signs devices (traditional, wearable or noncontact), beds, infusion pumps, point of care (POC) lab tests, etc.

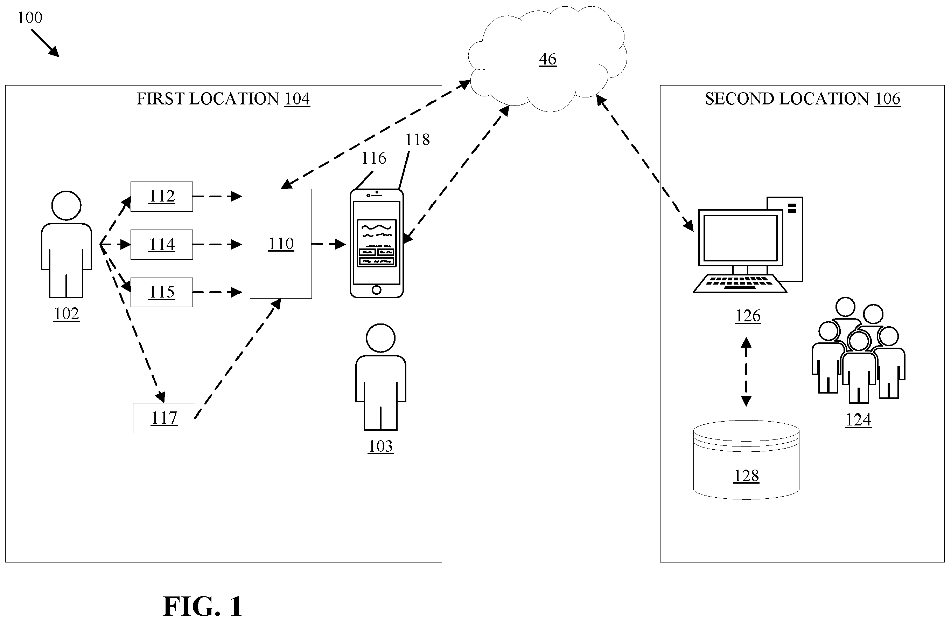

FIG. 1 is a schematic diagram of a system 100 that receives data from various medical devices in a care location.

The system 100 includes a patient 102 in a first location 104, such as a hospital room or clinic. The system 100 uses one or more devices 112, 114, 115 in the first location 104 to collect information from the patient 102.

For example, continuous or semi-continuous vital signs data can be collected from a medical device 112. The vital signs data obtained from the medical devices 112 can include any one or more of the following: heart rate data, respiration rate data, temperature data, pulse oximetry data, blood pressure data (including systolic and diastolic blood pressure), and the like. In one example, the medical device 112 is a Connex® Spot Monitor from Welch Allyn, Inc. of Skaneateles Falls, New York.

In some embodiments, the medical device 114 obtains patient movement data. The medical device 114 can, for example, be associated with a patient support device, such as the Centrella® Smart+ bed, Progressa® bed system, or VersaCare® Med Surg Bed, each available from Hillrom.

In further embodiments, the medical device 115 associated with the patient 102 collects clinical parameters, such as blood glucose level.

In some embodiments, the medical devices 112, 114, 115 can include a specialized vital signs patch (VSP) that is wearable by the patient 102 to obtain the medical data. In some embodiments, the one or more medical devices 112, 114, 115 include consumer grade devices such as wearable devices that incorporate fitness tracking and health-oriented capabilities including wearable activity trackers and smartwatches. In some embodiments, the one or more medical devices 112, 114, 115 are medical grade devices cleared by the Food and Drug Administration (FDA). In further embodiments, the one or more medical devices 112, 114, 115 include ambulatory electrocardiography devices such as a Holter monitor for cardiac monitoring for a given period of 24 to 48 hours.

Still referring to FIG. 1, the first location 104 includes a gateway device 110 that connects to each of the medical devices 112, 114, 115. In these examples, the gateway device 110 connects to the other medical device 112, 114, 115 using a wireless radio with a communication scheme such as WiFi®, Bluetooth®, near-field communication (NFC), radio frequency (RF) and the like. In some embodiments, the gateway device 110 can be embedded in one of the medical devices 112, 114, 115. For example, the gateway device 110 could be part of the vital signs monitoring device.

In this example, the gateway device 110 continuously (or periodically) scans the first location 104 to identify when a medical device is introduced into the first location 104. Upon a new medical device 117 being introduced, the gateway device 110 connects to the new medical device 117 wirelessly, identifies the new medical device 117, and notifies a caregiver 103 of the new medical device 117 in the first location 104 on a device 116, such as a smartphone.

For example, the gateway device 110 can use known wireless standards, such as Bluetooth Low Energy (BLE) to scan the first location 104 continuously to look for any new devices that enter the first location 104 and are also discoverable using BLE. Once the new medical device 117 is found by the gateway device 110, the gateway device 110 negotiates with the new medical device 117 using the standards set by BLE. For example, various profiles can be leveraged by BLE to facilitate the communications between the gateway device 110 and the new medical device 117, such as:
- BLP (Blood Pressure Profile) - for blood pressure measurement;
- HTP (Health Thermometer Profile) - for medical temperature measurement devices; and
- GLP (Glucose Profile) - for blood glucose monitors.

Other profiles for different types of devices and sensors can also be used, such as an ECG profile for communicating with electrocardiogram devices, a pump profile for communicating with other medical pumps, a ventilator profile for communicating with a ventilator, a video profile for communicating with various imaging devices, and an audio profile for communicating with various audio devices.

BLE is just one example of a wireless protocol that can be used. Other similar protocols, such as ANT, Bluetooth, WiFi, and Zigbee could also be used. Proprietary wireless protocols, such as the RTLS developed by Hillrom, can also be used.

Once a connection is made between the new medical device 117 and the gateway device 110, the gateway device 110 can present to the caregiver 103 information associated with the new medical device 117, allow the caregiver 103 to associate the new medical device 117 with the patient 102 so that medical data can be stored, and can present various algorithms and/or visualizations associated with the new medical device 117 that can be shown by an application 118 running on the device 116. This allows the new medical device 117 to be easily provisioned and automates the collection of medical data from the new medical device 117 so that the medical data can be stored and acted upon in a timely manner.

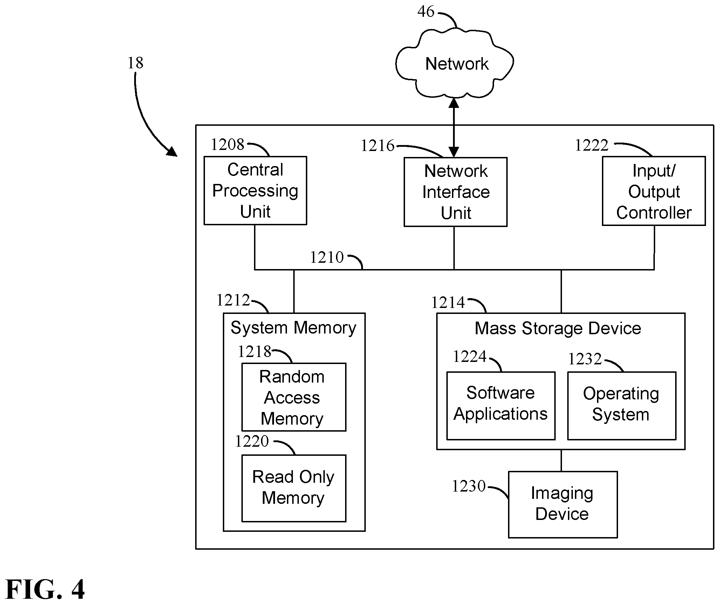

The gateway device 110 includes a computing device (described in reference to FIG. 4) having at least one processor and a memory. Stored in the memory of the gateway device 110 is an application 118 that connects via a network 46 to a remote computing device 126 at a second location 106, such as a data repository located at a different place in the hospital or clinic or offsite. For example, the remote computing device 126 can communicate with an electronic medical record (EMR) system 128 that stores medical data from the medical device 112, 114, 115.

The remote computing device 126 can be accessed by the caregiver 103, as well as other caregivers 124 at the second location 106 or located at other remote locations. As shown in FIG. 1, remote computing device 126 at the second location 106 can include one or more terminals, such as desktop computers, tablet computers, smartphones, and the like.

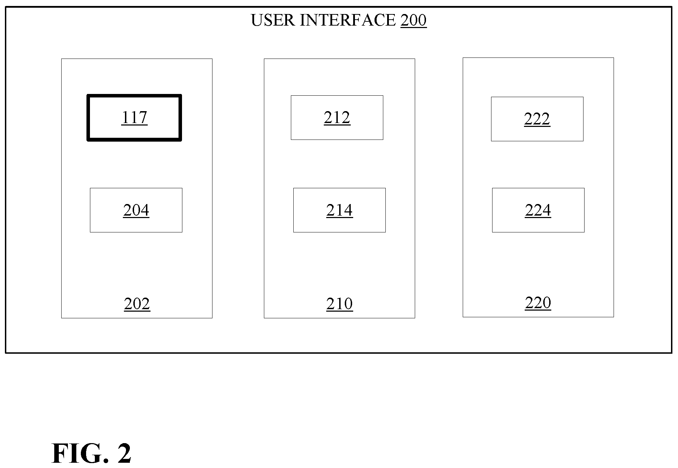

FIG. 2 is an example user interface 200 that is presented to the caregiver 103 (e.g., on the device 116) when the new medical device 117 is identified by the gateway device 110. In this example, the user interface 200 is generated by the application 118 running on the device 116. The user interface 200 gives the caregiver various options to connect and provision the new medical device 117.

For example, the user interface 200 includes a device selection section 202 that lists all medical devices that are available within the first location 104 which have not already been connected to the gateway device 110. In this example, the interface lists the new medical device 117 and another medical device 204. The caregiver 103 can simply select any or all of the medical devices by clicking or touching the desired medical devices. For example, the caregiver 103 can touch the new medical device 117 in the device selection section 202 to connect the new medical device 117 to the gateway device 110.

Once the new medical device 117 is selected, the user interface 200 provides an algorithm provisioning section 210. The algorithm provisioning section 210 provides different algorithm options 212, 214 that control how the new medical device 117 functions. The caregiver 103 selects one or more of the algorithm options 212, 214 to provision the new medical device 117.

For example, the algorithms can configure how the new medical device operates. This can include such parameters as how readings are taken, how often readings are taken, where and when data is sent, and other parameters specific to each new medical device. Other examples include configuring early warning scores and risk scores.

Further, the user interface 200 provides a data visualization section 220. The data visualization section 220 provides different visualization options 222, 224 that control how the data obtained by the new medical device 117 is displayed to the caregiver 103 on the device 116. The caregiver 103 selects one or more of the visual options 222, 224 to determine how the data is visualized.

For example, the visualization options can include how the data is presented, such as in tabular or graphical formats. It can include how much data is presented, such as by time duration or amount of data displayed. It can also include what data is presented and/or configure when data is shown (e.g., provide upper and/or lower limits).

Once the caregiver 103 has selected the desired visualization options, these options are stored so that when the caregiver 103 enters the first location 104 in future visits, the data is visualized on the device 116 in the manner desired.

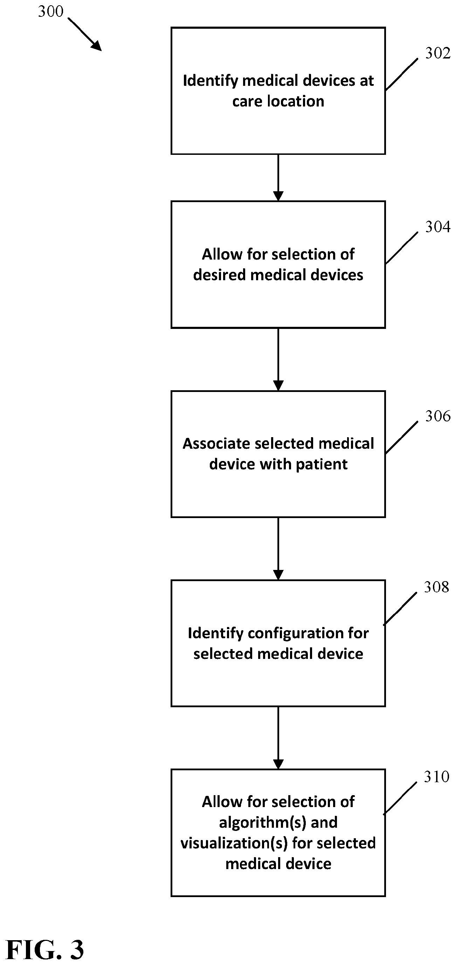

FIG. 3 illustrates an example method 300 of configuring and acquiring data from medical devices within a location. In this example, the caregiver orders periodic blood pressure measurements for the patient due to cardiac abnormalities. A blood pressure device is brought into the patient's room to take periodic measurements of the patient.

At operation 302, when the blood pressure device is brought into the room and powered on, the blood pressure device communicates with the gateway device using a communication scheme like BLE. The gateway device identifies the blood pressure device and notifies the caregiver of the presence of the blood pressure device.

Next, at operation 304, the caregiver can select the blood pressure device to connect the blood pressure device to the network, such as the EMR. The blood pressure device can be selected on an interface generated by the gateway device that lists all of the medical devices in the patient's room. See FIG. 2. In some configurations, the blood pressure device can be programmed to communicate directly with the EMR. In other configurations, the blood pressure device is programmed to communicate with the gateway device, and the gateway device, in turn, communicates data to and from the EMR.

Next, at operation 306, the blood pressure device is associated with the patient so that data captured by the blood pressure device can be automatically saved in the patient's record at the EMR system.

The association process can be automatic. For example, the gateway device can already be provisioned with the patient's information (e.g., name, patient identifier, etc.) so that the patient is automatically associated with the blood pressure device when the caregiver selects the blood pressure device. In another automated process, patient information is pulled from a remote system, such as an admit, discharge, and transfer (ADT) system, and that patient information is associated with the data. See U.S. Patent Application No. 62/786118 filed on December 28, 2018.

The association can also be manual. For example, once the caregiver selects the blood pressure device, the interface can allow the caregiver to manually enter the patient's information (e.g., name, patient identifier, etc.) so that the data captured by the blood pressure device is associated with the patient correctly. Many other configurations are possible.

Next, at operation 308, the configurations for the blood pressure device are identified. For example, the gateway device can query a data repository and/or come pre-configured with different configuration parameters associated with the blood pressure device. For example, the gateway device can look-up configuration information from a manufacturer of the medical device based upon unique identifiers associated with the blood pressure device, such as model number or serial number.

In another example, the blood pressure device can communicate configuration information directly to the gateway device. The configuration information can include such information as the algorithms used to control the blood pressure device. Further, the configuration information can include visualization data for the blood pressure device that defines how the data is presented to the caregiver.

Finally, at operation 310, the caregiver can select the desired configuration parameters for the blood pressure device. These can include the algorithms that control the blood pressure device. For example, the time periods between blood pressure measurements, alarm parameters, etc., can be configured. Also, the caregiver can select how the data is visualized. For example, the caregiver can select whether the data is presented in tabular or graphical format, how often the data is updated, and how much data is presented. Other configurations are possible.

FIG. 4 illustrates example physical components of a computing device associated with the devices described above, including the medical devices 112, 114, 115, the gateway device 110, the device 116, and/or the remote computing device 126. As illustrated, the computing device includes at least one processor or central processing unit ("CPU") 1208, a system memory 1212, and a system bus 1210 that couples the system memory 1212 to the CPU 1208. The system memory 1212 includes a random access memory ("RAM") 1218 and a read-only memory ("ROM") 1220. A basic input/output system containing the basic routines that help to transfer information between elements within the computing device, such as during startup, is stored in the ROM 1220. The computing device further includes a mass storage device 1214 able to store software instructions and data. The central processing unit 1208 is an example of a processing device.

The mass storage device 1214 is connected to the CPU 1208 through a mass storage controller (not shown) connected to the system bus 1210. The mass storage device 1214 and its associated computer-readable data storage media provide non-volatile, non-transitory storage for the computing device. Although the description of computer-readable data storage media contained herein refers to a mass storage device, such as a hard disk or CD-ROM drive, it should be appreciated by those skilled in the art that computer-readable data storage media can be any available non-transitory, physical device or article of manufacture from which the device can read data and/or instructions. The mass storage device 1214 is an example of a computer-readable storage device.

Computer-readable data storage media include volatile and non-volatile, removable and non-removable media implemented in any method or technology for storage of information such as computer-readable software instructions, data structures, program modules or other data. Example types of computer-readable data storage media include, but are not limited to, RAM, ROM, EPROM, EEPROM, flash memory or other solid-state memory technology, CD-ROMs, digital versatile discs ("DVDs"), other optical storage media, magnetic cassettes, magnetic tape, magnetic disk storage or other magnetic storage devices, or any other medium which can be used to store the desired information and which can be accessed by the computing device.

According to various embodiments, the computing device may operate in a networked environment using logical connections to remote network devices through the network 46, such as a local network, the Internet, or another type of network. The computing device connects to the network 46 through a network interface unit 1216 connected to the system bus 1210. The network interface unit 1216 may also be utilized to connect to other types of networks and remote computing systems. The computing device also includes an input/output controller 1222 for receiving and processing input from a number of other devices, including a camera, a keyboard, a mouse, a touch user interface display screen, or another type of input device. Similarly, the input/output controller 1222 may provide output to a touch user interface display screen, a printer, or other type of output device.

The computing device may also include an optional imaging device 1230, such as a camera that is configured to capture still or moving images (i.e., video). The camera can be configured to capture high resolution images or video (e.g., 100-200+ fps) that can be used to conduct one or more of the analyses described herein.

As mentioned above, the mass storage device 1214 and the RAM 1218 of the device can store software instructions and data. The software instructions include an operating system 1232 suitable for controlling the operation of the device. The mass storage device 1214 and/or the RAM 1218 also store software instructions, that when executed by the CPU 1208, cause the computing device to provide the functionality discussed in this document.

The use of the terms "a" and "an" and "the" and similar referents in the context of describing the subject matter (particularly in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. Furthermore, the foregoing description is for the purpose of illustration only, and not for the purpose of limitation, as the scope of protection sought is defined by the claims as set forth hereinafter together with any equivalents thereof entitled to.

The use of any and all examples, or exemplary language (e.g., "such as") provided herein, is intended merely to better illustrate the subject matter and does not pose a limitation on the scope of the subject matter unless otherwise claimed. The use of the term "based on" and other like phrases indicating a condition for bringing about a result, both in the claims and in the written description, is not intended to foreclose any other conditions that bring about that result.

Finally, as will be appreciated, any method aspects described herein may be encoded in instructions and stored on memory in the gateway device, such that the processor of the gateway device is caused to put those method aspects into effect.

## Claims

1. A gateway device for connecting a medical device to an electronic medical record, the system comprising:
a wireless radio to connect with a medical device positioned within a location of a patient;
a processor; and
memory encoding instructions which, when executed by the processor, cause the gateway device to:
allow a caregiver to select the medical device for connection;
identify a type of the medical device;
associate the medical device with the patient;
facilitate delivery of medical data from the medical device to the electronic medical record associated with the patient using the wireless radio;
present a plurality of algorithms for configuring the medical device to the caregiver for selection; and
present a plurality of data visualizations for visualizing the medical data captured by the medical device to the caregiver for selection.

2. The gateway device of claim 1, wherein the memory encodes further instructions which, when executed by the processor, cause the gateway device to display an interface listing medical devices available for connection with the gateway device.

3. The gateway device of claim 1 or 2, wherein the memory encodes further instructions which, when executed by the processor, cause the gateway device to display an algorithm provisioning section upon selection of one of the medical devices, the algorithm provisioning section showing the plurality of algorithms for configuring the medical device.

4. The gateway device of claim 1, 2 or 3, wherein the memory encodes further instructions which, when executed by the processor, cause the gateway device to display a data visualization section that shows the plurality of data visualizations.

5. The gateway device of any of claims 1 to 4, wherein the gateway device is a vital signs monitor.

6. The gateway device of any of the preceding claims, wherein the gateway device is part of a real-time location system.

7. The gateway device of any of the preceding claims, wherein the memory encodes further instructions which, when executed by the processor, cause the gateway device to automatically scan to identify the medical device.

8. The gateway device of claim 7, wherein the memory encodes further instructions which, when executed by the processor, cause the gateway device to notify the caregiver of the medical device.

9. A method for connecting a medical device to an electronic medical record, the method comprising:
displaying an interface listing medical devices available for connection with the gateway device;
allowing a caregiver to select the medical device for connection;
identifying a type of the medical device;
associating the medical device with the patient;
facilitate delivery of medical data from the medical device to the electronic medical record associated with the patient;
presenting a plurality of algorithms for configuring the medical device to the caregiver for selection; and
presenting a plurality of data visualizations for visualizing the medical data captured by the medical device to the caregiver for selection.

10. The method of claim 9, further comprising displaying an algorithm provisioning section upon selection of one of the medical devices, the algorithm provisioning section showing the plurality of algorithms for configuring the medical device.

11. The method of claim 9 or 10, further comprising displaying a data visualization section that shows the plurality of data visualizations.

12. The method of claim 9, 10 or 11, further comprising automatically scanning to identify the medical device.

13. The method of claim 12, further comprising notifying the caregiver of the medical device.

14. The method of any of claims 9 to 13, further comprising presenting one or more controls for defining the data visualizations.

15. The method of claim 14, further comprising presenting the data according to the data visualizations defined by the controls when the caregiver uses the medical device at a future visit.
